# EUROPEAN PATENT APPLICATION

(11) **EP 3 667 675 A1**
(43) Date of publication of application: **17.06.2020**
(21) Application number: 18211947.9
(22) Date of filing: 12.12.2018
(51) Int. Cl.: G16H 40/63, A61G 13/02, G05B 19/4061

(54) **MEDICAL APPARATUS AND METHOD FOR OPERATING THE MEDICAL APPARATUS**

(71) Applicant: TRUMPF Medizin Systeme GmbH + Co. KG, 07318 Saalfeld (DE)
(72) Inventor: HEMPEL, Felix, East Batesville, IN Indiana 47006 (US)
(74) Representative: Prüfer & Partner mbB Patentanwälte · Rechtsanwälte

(57) **Abstract**

A medical apparatus comprises a first component (2), at least one second component (4, 4', 4") movable with respect to the first component (2), a driving device (6, 6', 6") to drive the at least one second component (4, 4', 4"), a sensor (7, 7', 7") to detect a position of the at least one second component (4, 4', 4"), and a controller (8) to retrieve stored data of at least one bounding box (10, 10', 10", 10"", 10""', 10""") of the at least one second component (4, 4', 4"), to calculate actual positions of the at least one bounding box (10, 10', 10", 10'", 10"", 10""', 10""") based on the position of the at least one second component (4, 4', 4") as transformed data, and to determine a collision between the at least one second component (4, 4', 4") and the first component (2) or a surface (3) supporting the first component (2) by the transformed data and further transformed data of a further bounding box (11, 11') representing the first component (2) or of a surface (3) supporting the first component (2).

## Description

The invention relates to a medical apparatus and a method for operating the medical apparatus, in particular to a medical apparatus and a method for operating the medical apparatus including driving devices for adjusting positions of the medical apparatus.

Medical apparatuses such as patient supporting devices, in particular operating tables, include motor-driven components. Therefore, when adjusting a position of the motor-driven component, there is a risk of collision with other apparatuses or with another component of the apparatus f an operator operating the operating table is not aware of this risk. Such a collision, however, has the risk of injuring a patient lying on the operating table, the operator or a third person close to the operating table and the risk of damaging the equipment.

In this context, document US 2016/0166856 A1 discloses a predictive collision avoidance for radiotherapy which is intended to avoid collision between a patient support device or a patient and a radiation therapy machine. This equipment includes a three-dimensional camera identifying a plurality of objects in a depth map and a computing device generating a three dimensional model for each one of the plurality of objects which is very expensive. However, in case that no other equipment is located close to the medical apparatus, there is no need to take care of avoiding any collision with another equipment and such an expensive equipment is not necessary and would constitute an investment in vain.

Therefore, the object of the invention is to provide a medical apparatus and a method for operating the medical apparatus which avoids collision between components of the medical apparatus and with a surface supporting the medical apparatus.

The object is achieved by a medical apparatus according to claim 1 and a method according to claim 9. Advantageous further developments are included in the dependent claims.

According to an aspect of the invention, a medical apparatus comprises a first component and at least one second component movable with respect to the first component. The first component and the second component can also be constituted of several elements which are rigidly attached to one another, e.g. segments of a tabletop of an operating table, so that all of the several elements of the second component are together movable with respect to all of the elements of the first component.

The medical apparatus further comprises a driving device configured to drive the at least one second component and a sensor configured to detect a position and an orientation of the at least one second component.

Furthermore, the medical apparatus comprises a controller configured to retrieve stored data of at least one bounding box of the at least one second component. The at least one bounding box is an object oriented virtual bounding box representing a cuboid box including the at least one entire second component or a portion of the at least one second component of the medical apparatus. The data can be stored in the controller or in a memory separate from the controller. The controller is further configured to calculate actual positions and orientations of the at least one bounding box based on the position and the orientation of the at least one second component as transformed data of the at least one bounding box and to determine a collision or an imminent collision between the at least one second component and a first component or the surface supporting the first component by means of the transformed data and of further transformed data of a further bounding box representing a further virtual cuboid box including the entire first component or a portion of the first component or of a surface supporting the first component.

By such a configuration of the medial apparatus, equipment already provided in the medical apparatus can be used for determining a collision or an imminent collision between several components of the medical apparatus or between one of the components of the medical apparatus and the surface supporting the medical apparatus. Therefore, no additional equipment is necessary and an economical solution is achievable. Furthermore, due to forming the object virtual oriented bounding box, compared to processing a 3D shape of a component detected by a 3D camera, a necessary computing performance is reduced so that a less expensive controller can be used.

According to an advantageous implementation of the medical apparatus, the controller is configured to determine the imminent collision by means of a method of calculation according to the separating axis theorem.

By using this method of calculation, two convex shapes do not overlap when an axis along which a projection of the two shapes does not overlap can be found. Therefore, compared to other methods, less calculation performance is necessary and real time processing is possible.

According to a further advantageous implementation of the medical apparatus, the controller is configured to calculate the transformed data of the at least one bounding box by means of a method of calculation according to the forward kinematic model.

When the transformed data of the bounding boxes are calculated by the method of calculation according to the forward kinematic model, coordinates of the bounding boxes and, therefore, also locations and orientations of bounding boxes movably connected to each other, can easily be transformed into another coordinate system.

According to a further advantageous implementation of the medical apparatus, the transformed data are data in a world coordinate system.

Due to a transformation of the transformed data into the world coordinate system, the position and orientation of the single bounding boxes can easily be compared.

According to a further advantageous implementation of the medical apparatus, the controller is configured to control the driving device such that a motion of the at least one second component is stopped when its imminent collision is determined.

By controlling the driving device such that the motion of the at least one second component is stopped when its imminent collision is determined, a potential collision can be determined ahead and collisions can be predicted and prevented.

According to a further advantageous implementation of the medical apparatus, the medical apparatus comprises several second components and the controller is configured to determine an imminent collision between anyone of the several second components and another one of the several second components.

When providing several second components, e.g., in the case of an operating table of the medical apparatus, a more sophisticated patient positioning is possible. Due the determination of the imminent collision also between the several second components, a safe use of the medical apparatus is possible.

According to a further advantageous implementation of the medical apparatus, the controller is configured to automatically detect the at least one second component and further components attached to the first component and to the at least one second component and to generate or adjust an internal physical model of the medical apparatus comprising the at least one bounding box and the further bounding box and/or by the surface supporting the first component.

By the automatic detection of the second components and the further components, an actual configuration of the medical apparatus can easily be determined. Even if a component consists of several elements, an appropriate bounding box or a set of bounding boxes of the component can be used so that the internal physical model for calculating the locations and orientations of the several components can be adjusted to the actual configuration. This is in order to determine imminent collisions of the second components between themselves, between one of the second components and the first component or between one of the second components and the surface supporting the first component.

According to a further advantageous implementation of the medical apparatus, the medical apparatus is configured as an operating table and the surface supporting the first component is a floor.

An operating table supported by the floor is a suitable application for the determination of the collision since, when a patient lying on the operating table is covered with surgery drapes, boundaries of a tabletop of the operating table cannot be recognized by an operator so that, due to the collision determination, a potential collision can be prevented.

According to a further aspect of the invention, a method for operating the medical apparatus is provided. The method includes the steps: defining and storing respective virtual bounding boxes for the first component and the at least one second component, calculating actual positions and orientations of the at least one bounding box of the at least one second component based on the position and the orientation of the at least one second component detected by the sensor as the transformed data of the at least one bounding box in the actual position and orientation of the at least one second component, and determining a collision between one of the at least one second component and another one of the at least one second component, the first component or the surface supporting the first component.

By using such a method, equipment already provided in the medical apparatus can be used for determining an imminent collision between several components of the medical apparatus or between one of the components of the medical apparatus and the surface supporting the medical apparatus. Therefore, no additional equipment is necessary and an economical solution is achievable.

In an advantageous implementation of the method, the collision is determined by a method of calculation according to the separating axis theorem.

By using this method of calculation, compared to other methods, less calculation performance is necessary and real time processing is possible.

In a further advantageous implementation of the method, the position and the orientation of the at least one bounding box of the at least one second component is calculated by using a method of calculation according to the forward kinematic model.

When the transformed data of the bounding boxes are calculated by the method of calculation according to the forward kinematic model, coordinates of the bounding boxes and, therefore, location and orientation also of bounding boxes movably connected to each other, can easily be transformed into another coordinate system.

In a further advantageous implementation of the method, the transformed data are calculated in a world coordinate system.

Due to a transformation of the transformed data into the world coordinate system, the position and orientation of the single bounding boxes can easily be compared.

In a further advantageous implementation of the method, the motion of the at least one concerned second component is stopped when its imminent collision is determined.

By stopping the motion when an imminent collision is determined from a motion of the bounding boxes with respect to each other, the potential collision can be determined ahead so that collisions can be predicted and prevented.

In a further advantageous implementation of the method, the at least one second component and further components attached to the first component and to the at least one second component are automatically detected to generate or adjust an internal physical model of the medical apparatus defined by the at least one bounding box and the further bounding box and/or the surface supporting the first component.

By the automatic detection of the second components and the further components, an actual configuration of the medical apparatus can easily be determined. Even if a component consists of several elements, an appropriate bounding box or a set of bounding boxes of the component can be used so that the internal physical model for calculating the locations and orientations of the several components can be generated or adjusted to the actual configuration in order to determine collision of the second components between themselves, between one of the second components and the first component or between one of the second components and the surface supporting the first component.

Below, the invention is elucidated by means of embodiments referring to the attached drawings.

In particular,
- Fig. 1: shows an embodiment of a medical apparatus according to the invention;
- Fig. 2: shows an internal physical model of the medical apparatus of another embodiment of the invention; and
- Fig. 3: shows a flowchart illustrating a method according to the invention.

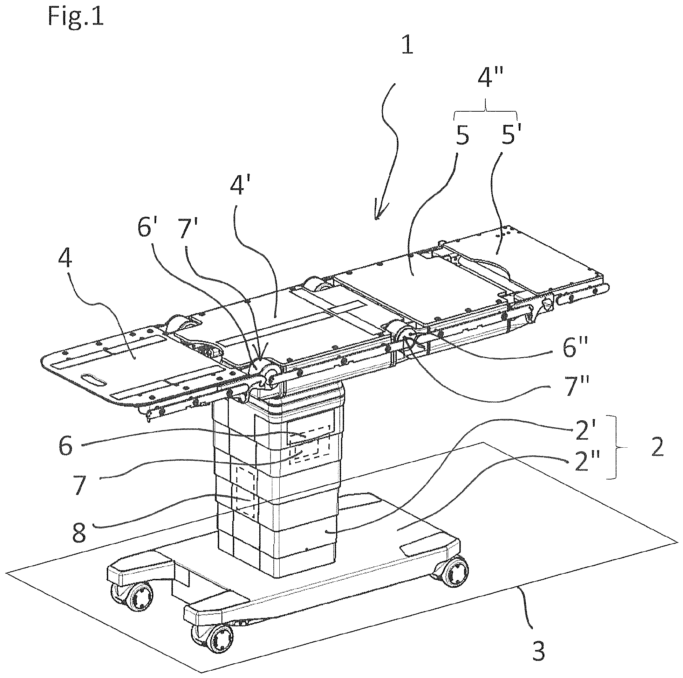

Fig. 1 shows an embodiment of a medical apparatus 1 according to the invention. The medical apparatus 1 is configured as an operating table. The operating table comprises a column 2' and a carriage 2" as a first component 2. The carriage 2" and, therefore, the first component 2, is supported by a floor. Hence, the floor is a surface 3 supporting the first component 2. In this embodiment, the first component 2 is illustrated as a component consisting of two elements, i.e., of the column 2' and the carriage 2" which are rigidly attached to another. However, in alternative embodiments, the first component 2 can be composed by a single element.

Furthermore, the operating table comprises several sections of a tabletop as second components 4, 4', 4" which are movable with respect to the first component 2. In this embodiment, the second components 4, 4', 4" are movable with respect to the first component 2 and also with respect to another one of the second components 4, 4', 4". The second components 4, 4' are constituted by a single element, wherein small accessories, such as accessory rails, are regarded as to be included in the single element. The second component 4"' is formed by two elements 5, 5' which are rigidly attached to one another. In alternative embodiments, at least one second component 4, 4', 4" movable with respect to the first component 2 is provided.

Moreover, the operating table comprises driving devices 6, 6', 6". The driving devices 6, 6', 6" respectively drive one of the second components 4, 4', 4" with respect to the first component 2. In particular, the driving device 6 moves the tabletop and, therefore, the second components 4, 4', 4" in a height-adjustable manner. The driving device 6' moves a leg section as the second component 4 with respect to the first component 2 and with respect to a seat section as the second component 4'. The driving device 6" moves a back section as the element 5 and a head section as the element 5' constituting the second component 4" with respect to the second component 4' and with respect to the first component 2. In alternative embodiments, at least one driving device 6, 6', 6" is provided.

The operating table is provided with sensors 7, 7', 7" for detecting a position and an orientation of the second components 4, 4', 4". The sensors 7, 7', 7" respectively detect a positon of one of the driving devices 6, 6', 6". However, in alternative embodiments, another type of sensors, e.g., a position sensor included in one of the tabletop sections, can be provided, wherein at least one sensor has to be provided.

Additionally, the operating table comprises a controller 8. The controller 8 controls the driving devices 6, 6', 6".

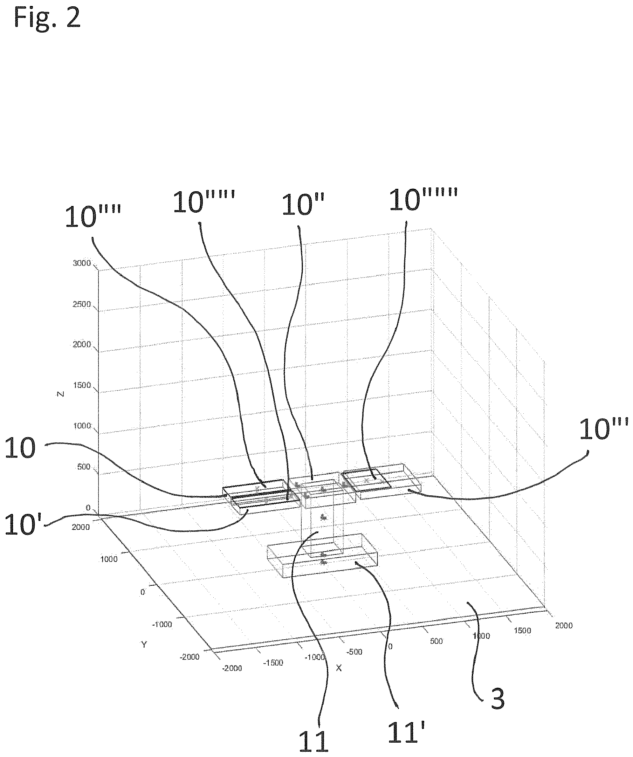

Fig. 2 shows an illustration of an internal physical model of the medical apparatus 1 of another embodiment of the invention. The embodiment of the operating table of Fig. 2 differs from the embodiment of the operating table of Fig. 1 in a modified leg section. In Fig. 1, the leg section is formed by one leg plate and, in Fig. 2, the leg section is formed by two parallel leg plates.

The internal physical model comprises bounding boxes 10, 10', 10", 10"', 10"", 10""', 10""" which are object oriented and which respectively correspond to the second components 4, 4', 4". Due to the different embodiments, the bounding boxes 10, 10' do not respectively include the entire second component 4 but, nevertheless, the bounding boxes 10, 10' are regarded as respectively including one of the two leg plates. The bounding box 10" includes an entire seat plate of the tabletop and the bounding box 10'" includes an entire back plate of the tabletop. The bounding boxes 10"", 10""', 10""" respectively include portions of the second components, i.e., a respective upper portion forming a lying surface of the leg plates and of the back plate. In alternative embodiments, other suitable portions of the components are included in the bounding boxes. Furthermore, the internal physical model comprises further bounding boxes 11, 11' which are object oriented and which correspond to the first component 2, in particular to the column 2' and the carriage 2" as portions of the first component 2. The bounding boxes 10, 10', 10", 10'", 10"", 10""', 10""" and further bounding boxes 11, 11' respectively represent a virtual cuboid box including the respective first component 2 or at least portions of one of the second components 4, 4', 4". The bounding boxes 10, 10', 10", 10'", 10"", 10""', 10""", 11, 11' are defined by the X, Y, and Z dimensions of the respective second component 4, 4', 4" and the bounding boxes 10, 10', 10", 10'", 10"", 10""', 10""" by a translation from the center of the bounding box to a rotation center in X, Y, Z direction.

The controller 8 is configured to retrieve stored data of the bounding boxes and further bounding boxes 10, 10', 10", 10'", 10"", 10""', 10""",11, 11' and of the surface 3 supporting the first component 2. The data are stored in the controller, however, in alternative embodiments, the data are stored in a memory separate from the controller. Furthermore, the controller 8 is configured to calculate actual positions and orientations of the bounding boxes 10, 10', 10", 10'", 10"", 10""', 10""" based on the position and the orientation of the second components detected by the sensors 7, 7', 7" as transformed data of the bounding boxes 10, 10', 10", 10'", 10"", 10""', 10""". Furthermore, actual positions of bounding boxes 11, 11' of the first component 2 and of the surface 3 supporting the first component 2 are calculated as further transformed data.

The controller 8 is further configured to calculate the transformed data of the actual positions and orientations of the bounding boxes 10, 10', 10", 10'", 10"", 10""', 10""" of the further bounding boxes 11, 11' and of the surface 3 supporting the first component 2 by a known method of calculation according to the forward kinematic model. The forward kinematic model refers to the use of kinematic equations of an apparatus having mobile components joined to one another to compute a position of an end component from specified values for joint parameters. The transformed data are data in a world coordinate system. The world coordinate system is a coordinate system, the origin and axis of which are defined by a user. This world coordinate system enables a control to use a common coordinate system for positions of several components of the medical apparatus. This method of calculation incorporates the entire three-dimensional model of the medical device 1, tolerances of the components, a kind of connection of the components, and the position and orientation with respect to each other. In an alternative embodiment, another suitable method of calculation can be executed, as, e.g. using quaternions or Jacobian matrices, and/or the transformed data are data in another suitable coordinate system, e.g., in a coordinate system of one of the bounding boxes.

Furthermore, the controller 8 is configured to determine an imminent collision between one of the second components 4, 4', 4" and the first component 2 or a surface 3 supporting the first component 2 by means of the transformed data and of the further transformed data of the further bounding box 11, 11' including the entire first component 2 or of a surface 3 supporting the first component 2. The collision is determined by a method of calculation according to the separating axis theorem. Correspondent to the method of calculation according to the separating axis theorem, basically, two convex shapes do not overlap when an axis along which a projection of the two shapes does not overlap can be found. In 3D, three normal of faces of a first bounding box, three normal of faces of a second bounding box and nine further normals of faces including opposite edges of the bounding boxes are to be checked. In an alternative embodiment, the collision is determined by means of another calculation method, as, e.g., the sweep and prune algorithm, surface mesh approaches, or voxel-based approaches.

The controller 8 is configured to control the driving devices 6, 6', 6" such that a motion of the second components 4, 4', 4" is stopped when the imminent collision is determined. The collision is determined when the bounding boxes of one of the second components 4, 4', 4" and of another one of the second components 4, 4', 4" or of the first component 2 or the surface 3 supporting the first component 2 intersect. In an alternative embodiment, an approach within specific circumstances is sufficient for determining the collision ahead.

Moreover, the controller 8 is configured to automatically detect the second components 4, 4', 4" and further components attached to the first component 2 and to the second components 4, 4', 4" and to generate or adjust the internal physical model of the medical apparatus 1 comprising the bounding boxes 10, 10', 10", 10'", 10"", 10""', 10""" and the further bounding boxes 11, 11'. The further components are additional sections of the tabletop or accessory parts rigidly attached to one of the second components 4, 4', 4" or the first component 2.

In alternative embodiments, the functions of storing und calculating are not performed by the controller 8 but by another computing unit connected to the controller 8.

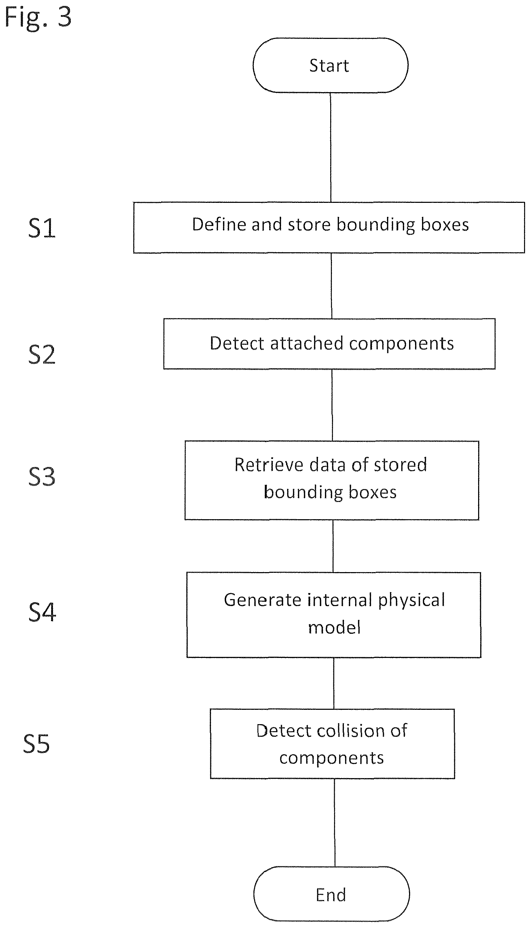

Fig. 3 shows a flowchart illustrating a method according to the invention. In use, geometric data of the components of the medical apparatus 1 are generated or already existing geometric data, e.g. from a CAD system, are provided. Subsequently, in step S1, cuboid boxes are defined and stored as bounding boxes 10, 10', 10", 10"', 10"", 10""', 10""", 11, 11' which include moving segments, i.e., the second components 4, 4', 4", and segments, the moving segments move with respect to, i.e. the first components 2, 2'.

Upon receiving new movement commands from an operator, in step S2, the second components 4, 4', 4" and further components attached to the first component 2, 2' and to the second components 4, 4', 4" are automatically detected. Thereby, the controller is able to generate or adjust an internal physical model of the medical apparatus 1 defined by the at least one bounding box 10, 10', 10", 10'", 10"", 10""', 10""" representing the second components 4, 4', 4" and the further bounding box 11, 11' representing the first component 2 and/or the surface 3 supporting the first component 2.

In step S3, then, the data of the defined bounding boxes 10, 10', 10", 10'", 10"", 10""', 10""", 11, 11' are retrieved by the controller.

In a next step S4, an actual motor position and sensor information are used to generate the internal physical model of the operating table in its actual state. Actual positions and orientations of the bounding boxes 10, 10', 10", 10'", 10"", 10""', 10""" of the second components 4, 4', 4" transformed into a world coordinate system are calculated as the transformed data of the bounding boxes in the actual position and orientation of the second components based on the position and the orientation of the second components 4, 4', 4" detected by the sensors 7, 7', 7". These calculations are performed by means of the known method of calculation according to the forward kinematic model. In an alternative embodiment, this calculation is performed by means of another method of calculation, as, e.g. using quaternions or Jacobian matrices, and/or the positions and orientations of the bounding boxes are transformed into another suitable coordinate system.

In step S5, an imminent collision between one of the second components and another one of the second components, the first component or the surface 3 supporting the first component 2 is determined. The determination of the collision is performed by the known method of calculation according to the separating axis theorem. In an alternative embodiment, the determination is performed by means of another method of calculation, as, e.g., the sweep and prune algorithm, surface mesh approaches, or voxel-based approaches.

When its imminent collision is determined, the motion of the concerned second component is stopped. The collision can be determined by a beginning intersection of the bounding boxes or, in advance, by a direction of an approach of the bounding boxes within specific circumstances. In alternative embodiments, instead of or additional to stopping the motion, a warning is issued.

While the invention has been illustrated and described in detail in the drawings and the foregoing description, such illustration and description are to be considered illustrative or exemplary and not restrictive. The invention is not limited to the disclosed embodiments. From reading the present disclosure, other modifications will be apparent to a person skilled in the art. Such modifications may involve other features, which are already known in the art and may be used instead of or in addition to features already described herein. In the claims, the word "comprising" does not exclude other elements or steps, and the indefinite article "a" or "an" does not exclude a plurality.

## Claims

1. A medical apparatus (1) comprising
a first component (2),
at least one second component (4, 4', 4") movable with respect to the first component (2),
at least one driving device (6, 6', 6") configured to drive the at least one second component (4, 4', 4"),
a sensor (7, 7', 7") configured to detect a position and an orientation of the at least one second component (4, 4', 4"), and
a controller (8) configured
to retrieve stored data of at least one bounding box (10, 10', 10", 10"', 10"", 10""', 10""") of the at least one second component (4, 4', 4"), the at least one bounding box (10, 10', 10", 10'", 10"", 10""', 10""") representing a virtual cuboid box including the at least one entire second component (4, 4', 4") or a portion of the at least one second component (4, 4', 4"),
to calculate actual positions and orientations of the at least one bounding box (10, 10', 10", 10'", 10"", 10""', 10""") based on the position and the orientation of the at least one second component (4, 4', 4") as transformed data of the at least one bounding box (10, 10', 10", 10'", 10"", 10""', 10"""), and
to determine an imminent collision between the at least one second component (4, 4', 4") and the first component (2) or a surface (3) supporting the first component (2) by means of the transformed data and further transformed data of a further bounding box (11, 11') representing a further virtual cuboid box including the entire first component (2) or a portion of the first component (2) or of a surface (3) supporting the first component (2).

2. The medical apparatus (1) of claim 1, wherein
the controller (8) is configured to determine the collision by means of a method of calculation according to the separating axis theorem.

3. The medical apparatus (1) of claim 1 or 2, wherein
the controller (8) is configured to calculate the transformed data of the at least one bounding box (10, 10', 10", 10"', 10"", 10""', 10""") by means of a method of calculation according to the forward kinematic model.

4. The medical apparatus (1) of anyone of the preceding claims, wherein
the transformed data are data in a world coordinate system.

5. The medical apparatus (1) of anyone of the preceding claims, wherein the controller (8) is configured to control the driving device (6, 6', 6") such that a motion of the at least one second component (4, 4', 4") is stopped when its imminent collision is determined.

6. The medical apparatus (1) of anyone of the preceding claims, wherein
the medical apparatus (1) comprises several second components (4, 4', 4"), and
the controller (8) is configured to determine an imminent collision between anyone of the several second components (4, 4', 4") and another one of the several second components (4, 4', 4").

7. The medical apparatus (1) of claim 6, wherein
the controller (8) is configured to automatically detect the at least one second component (4, 4', 4") and further components attached to the first component (2) and to the at least one second component (4, 4', 4") and to generate or adjust an internal physical model of the medical apparatus (1) comprising the at least one bounding box (10, 10', 10", 10'", 10"", 10""', 10""") and the further bounding box (11, 11') and/or by the surface (3) supporting the first component (2).

8. The medical apparatus (1) of anyone of the preceding claims,
wherein the medical apparatus (1) is configured as an operating table and the surface (3) supporting the first component (2) is a floor.

9. Method for operating a medical apparatus (1) of anyone of the preceding claims including the steps:
defining respective virtual bounding boxes (10, 10', 10", 10"', 10"", 10""', 10""", 11, 11') for the first component (2) and the at least one second component (4, 4', 4");
storing the respective bounding boxes (10, 10', 10", 10'", 10"", 10""', 10""", 11, 11') for the first component and the at least one second component (4, 4', 4");
calculating actual positions and orientations of the at least one bounding box (10, 10', 10", 10'", 10"", 10""', 10""") of the at least one second component (4, 4', 4") based on the position and the orientation of the at least one second component (4, 4', 4") detected by the sensor (7, 7', 7") as the transformed data of the at least one bounding box (10, 10', 10", 10'", 10"", 10""', 10""") in the actual position and orientation of the at least one second component (4, 4', 4"); and
determining an imminent collision between one of the at least one second component (4, 4', 4") and another one of the at least one second component (4, 4', 4"), the first component (2) or the surface (3) supporting the first component (2).

10. The method of claim 9, wherein
the imminent collision is determined by a method of calculation according to the separating axis theorem.

11. The method of claim 9 or 10, wherein
the position and the orientation of the at least one bounding box (10, 10', 10", 10'", 10"", 10""', 10""") of the at least one second component (4, 4', 4") is calculated by using a method of calculation according to the forward kinematic model.

12. The method of anyone of claims 9 to 11, wherein the transformed data are calculated in a world coordinate system.

13. The method of anyone of claims 9 to 12, wherein
a motion of the at least one concerned second component (4, 4', 4") is stopped when its imminent collision is determined.

14. The method of anyone of claims 9 to 13, wherein
the at least one second component (4, 4', 4") and further components attached to the first component (2) and to the at least one second component (4, 4', 4") are automatically detected to generate or adjust an internal physical model of the medical apparatus (1) defined by the at least one bounding box (10, 10', 10", 10"', 10"", 10""', 10""") and the further bounding box (11, 11') and/or the surface (3) supporting the first component (2).
